(19) Europäisches Patentamt
European Patent Office
Office européen des brevets

(11) Veröffentlichungsnummer: **0 175 983**
**B1**

(12) **EUROPÄISCHE PATENTSCHRIFT**

(45) Veröffentlichungstag der Patentschrift: **02.08.89**

(51) Int. Cl.⁴: **C 07 C 147/06**, C 07 C 161/05, C 07 F 9/09

(21) Anmeldenummer: **85111255.7**

(22) Anmeldetag: **06.09.85**

(54) **4,4'-Bis-(beta-hydroxyethylsulfonyl)-diphenyl und dessen Ester sowie Verfahren zu ihrer Herstellung.**

(30) Priorität: **15.09.84 DE 3433981**

(43) Veröffentlichungstag der Anmeldung: **02.04.86 Patentblatt 86/14**

(45) Bekanntmachung des Hinweises auf die Patenterteilung: **02.08.89 Patentblatt 89/31**

(84) Benannte Vertragsstaaten: **CH DE FR GB LI SE**

(56) Entgegenhaltungen: --

(73) Patentinhaber: **HOECHST AKTIENGESELLSCHAFT, Postfach 80 03 20, D-6230 Frankfurt am Main 80 (DE)**

(72) Erfinder: **Springer, Hartmut, Dr., Am Erdbeerstein 27, D-6240 Königstein/Taunus (DE)**
Erfinder: **König, Gerd, Dr., Breckenheimer Strasse 32, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 175 983 B1

LIBER, STOCKHOLM 1989

**Beschreibung**

Mit der vorliegenden Erfindung wurden neue Verbindungen gefunden, die sich zur Modifizierung und Verbesserung der Eigenschaften von Wolle und anderen carbonamidgruppenhaltigen Materialien eignen. Die neuen Verbindungen besitzen die allgemeine Formel (1)

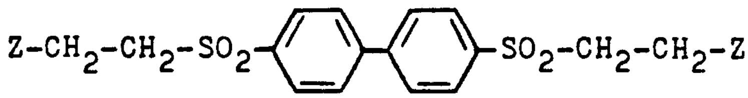

(1)

in welcher beide Z bevorzugt zueinander gleiche Bedeutungen besitzen und jedes eine Hydroxygruppe oder eine Sulfatogruppe (entsprechend der allgemeinen Formel $-OSO_3M$ , in welcher M für ein Wasserstoffatom oder ein Alkalimetall, wie Natrium, Kalium, Lithium, oder für das Äquivalent eines Erdalkalimetalls, wie des Calciums, steht) oder eine Thiosulfatogruppe (entsprechend der allgemeinen Formel $-S-SO_3M$ mit M der obengenannten Bedeutung) oder eine Phosphatogruppe (entsprechend der allgemeinen Formel $-OPO_3M_2$ mit M der obengenannten Bedeutung) ist. Erfindungsgemäße Verbindungen der allgemeinen Formel (1), in denen ein oder beide Z für eine Hydroxygruppe stehen, dienen als Vorprodukte für die Herstellung der erfindungsgemäßen Verbindungen (1), in denen die Z jeweils eine Sulfato-, Thiosulfato- oder Phosphatogruppe sind.

Bevorzugt sind diejenigen erfindungsgemäßen Verbindungen, in denen eines oder bevorzugt beide Z jeweils eine Sulfatogruppe bedeuten.

Die vorliegende Erfindung betrifft weiterhin ein Verfahren zur Herstellung dieser Verbindungen der allgemeinen Formel (1). Dieses ist dadurch gekennzeichnet, daß man Diphenyl sulfochloriert, das Diphenyl-4,4'-disulfochlorid mit der wäßrigen Lösung eines Alkali- oder Erdalkalihydrogensulfits oder -sulfits umsetzt und auf diese Weise die Sulfochloridgruppen in Sulfinsäuregruppen bzw. deren Alkalisalze oder Erdalkalisalze überführt, sodann die beiden Sulfinsäuregruppen mit Ethylenoxid zu den β-Hydroxyethylsulfonyl-Gruppen umsetzt und das 4,4'-Bis-(β-hydroxyethylsulfonyl)-diphenyl gegebenenfalls zur Herstellung von dessen Schwefelsäure- oder Phosphorsäurehalbester (Sulfato- bzw. Phosphatoverbindungen) mit einem Sulfatierungs- bzw. Phosphatierungsmittel umsetzt, und gegebenenfalls zur Herstellung der Thiosulfatoverbindungen der Formel (1) die Sulfatoverbindung der Formel (1), in welcher Z für die Sulfatogruppe(n) steht, mit einem Alkalithiosulfatsalz in wäßriger Lösung in zunächst alkalischem und dann schwach sauren Bereich umsetzt.

Die Methoden der Sulfochlorierung, der Reduktion zur Sulfinsäuregruppe, der Oxethylierung, der Sulfatierung und der Phosphatierung sowie der Überführung von β-Sulfatoethylsulfonyl-Gruppen in β-Thiosulfatoethylsulfonyl-Gruppen sind an und für sich bekannt. Die erfindungsgemäßen Umsetzungen können analog solchen in der Literatur beschriebenen Verfahrensweisen erfolgen.

Die Sulfochlorierung erfolgt beispielsweise mit Chlorsulfonsäure oder Monohydrat und Thionylchlorid; sie kann analog einer Verfahrensweise, wie sie beispielsweise in Houben-Weyl, Methoden der Organischen Chemie, Band IX, S. 578 (1955), beschrieben ist, durchgeführt werden, wobei man vorzugsweise bei einer Temperatur zwischen 40 und 100°C, insbesondere zwischen 60 und 70°C arbeitet.

Das Diphenyl-4,4'-disulfochlorid wird sodann, beispielsweise analog zu Houben-Weyl, loc. cit., S. 306, bevorzugt mittels einem Alkalisulfit oder Alkalihydrogensulfit, insbesondere Natriumsulfit oder Natriumhydrogensulfit, in Gegenwart eines Alkalicarbonats oder Alkalihydroxids, wie Natriumcarbonat oder Natriumhydroxid, in wäßriger Lösung, vorzugsweise bei einer Temperatur zwischen 0°C und 80°C, insbesondere zwischen 60 und 70°C, und bei einem pH-Wert zwischen 7,0 und 10,0, bevorzugt zwischen 8,0 und 8,5 zu der entsprechenden Diphenyl-4,4'-disulfinsäure reduziert. Diese wird sodann mit Ethylenoxid in Gegenwart von wäßriger Schwefelsäure oder Phosphorsäure, beispielsweise analog der in der US-Patentschrift Nr. 3 647 813 beschriebenen Verfahrensweise, bei einer Temperatur zwischen 30 und 70°C, vorzugsweise zwischen 50 und 60°C, und bei einem pH-Wert zwischen 4,5 und 9,0, insbesondere zwischen 7,0 und 8,0, zur erfindungsgemäßen Verbindung der allgemeinen Formel (1), in welchen beide Z für die Hydroxygruppe stehen, d.h. zum 4,4'-Bis-(β-hydroxyethylsulfonyl)-diphenyl, umgesetzt.

Die Veresterung der β-Hydroxyethylsulfonyl-Verbindung in deren Sulfato- bzw. Phosphatoderivate kann analog Verfahrensweisen erfolgen, die beispielsweise in den deutschen Auslegeschriften Nrs. 1 248 188, 1 256 542 und 1 443 877 bzw. in der deutschen Auslegeschrift 1 795 086 beschrieben sind. Als Sulfatierungsmittel dienen beispielsweise 98 bis 100-%-ige Schwefelsäure oder Schwefeltrioxid enthaltende Schwefelsäure oder Chlorsulfonsäure oder Amidosulfonsäure in Gegenwart von Pyridin; als Phosphatierungsmittel können Phosphorsäure bzw. Polyphosphorsäure oder Lösungen von Phosphorpentoxid in Phosphorsäure bzw. Polyphosphorsäure verwendet werden. So kann man zur Herstellung der erfindungsgemäßen β-Sulfatoethylsulfonyl-Verbindung aus der entsprechenden β-Hydroxyethylsulfonyl-Verbindung diese Hydroxyverbindung mit 98 bis 100-%-iger Schwefelsäure oder etwa bis zu 30 Gew.-% Schwefeltrioxid enthaltender Schwefelsäure bei einer Temperatur zwischen -5°C und +50°C, vorzugswsise zwischen 20 und 25°C, sulfatieren. Die gebildete Sulfatoverbindung kann aus ihrer mit Eis verdünnten sauren Lösung bei Raumtemperatur oder einer niedrigeren Temperatur ausgefällt und isoliert werden; die erhaltene saure Verbindung kann sodann in üblicher Weise mittels einem Alkalihydroxid, Alkalicarbonat oder Alkalihydrogencarbonat oder einem Erdalkalihydroxid oder -carbonat in deren Salze übergeführt und aus dem wäßrigen Medium durch Sprühtrocknung isoliert werden.

Die Umsetzung der erfindungsgemäßen Hydroxyethylsulfonyl-Verbindung in deren β-Phosphatoethylsulfonyl-Derivate erfolgt mit den genannten Phosphatierungsmitteln beispielsweise bei einer Temperatur zwischen 20 und 120°C, vorzugsweise bei einer Temperatur zwischen 95 und 100°C. Wie die sauren Sulfatoverbindungen können die sauren Phosphatoverbindungen in deren Salze überführt und isoliert werden.

Die erfindungsgemäßen Thiosulfato-Verbindungen lassen sich beispielsweise analog den Verfahrensweisen herstellen, wie sie beispielsweise in der deutschen Patentschrift 1 246 906 beschrieben sind. So verfährt man zur Herstellung der erfindungsgemäßen Thiosulfatoverbindungen bevorzugt in der Weise, daß man die entsprechende erfindungsgemäße Sulfatoverbindung in wäßriger Lösung mittels einem Alkalihydroxid- oder -carbonat bei einem pH-Wert zwischen 10 und 13, vorzugsweise zwischen 11,5 und 12,3, bei einer Temperatur zwischen 20 und 80°C, vorzugsweise zwischen 35 und 45°C, in die entsprechende Vinylsulfonyl-Verbindung überführt. Die wäßrige Lösung oder Suspension der Vinylsulfonyl-Verbindung wird sodann mit einem Alkalithiosulfat, bevorzugt Natriumthiosulfat, bei einer Temperatur zwischen 40 und 90°C, bevorzugt zwischen 70 und 75°C, und bei einem pH-Wert zwischen 4,5 und 7,5, bevorzugt zwischen 5,7 und 6,2, zur Lösung der β-Thiosulfatoethylsulfonyl-Verbindung umgesetzt. Die Thiosulfatoverbindung kann durch Zugabe von Alkalihalogeniden, bevorzugt Kaliumchlorid, aus der wäßrigen Lösung ausgefällt und isoliert werden.

Die neuen Verbindungen der allgemeinen Formel (1) dienen zur Modifizierung und Verbesserung der physikalischen und färberischen Eigenschaften von Materialien aus natürlichen und/oder synthetischen Polyamidfasern, wie Wolle, Polyamid-6, Polyamid-6,6 und Polyamid-11, und/oder Polyurethanfasern sowie als wertvolle Hilfsmittel in Färbeprozessen für solche Materialien. So verbessern sie beispielsweise die Egalität von Polyamidfärbungen, wenn man sie vor dem eigentlichen Färbeprozeß oder zusammen mit dem Farbstoff während des Färbeprozesses auf die natürliche und/oder synthetische Polyamidfaser einwirken läßt. In gleicher Weise gelingt es beispielsweise, die Naßreißfestigkeit von Wolle durch Behandlung mit den neuen Verbindungen der Formel (1) zu verbessern. Auch kann der Faserverlust, der üblicherweise beim Färben von Wolle bei Kochtemperatur auftritt, mit Hilfe der neuen Verbindungen der Formel (1) vermindert werden. Denn beim Färben von Wolle wird infolge der Färbebedingungen ein Teil der Wolle durch Hydrolyse zerstört. Dies äußert sich in reduzierter Faserfestigkeit sowie in erhöhter Alkalilöslichkeit der Wolle. Ähnliches gilt in gewissem Umfange für Polyamid- und Polyurethanfasermaterialien. Die Behandlung von Wolle bzw. dieser synthetischen Materialien mit einer erfindungsgemäßen Verbindung in einem wäßrigen Bad bewirkt allerdings, daß die Beständigkeit von Wolle und ebenso der anderen carbonamidgruppenhaltigen Fasern gegenüber den erforderlichen Färbebedingungen erheblich verbessert wird. Der Verlust an Festigkeit nach der Färbung wird ebenso wie die Alkalilöslichkeit verringert. Desweiteren tritt eine Verbesserung der Naßechtheitseigenschaften der erhaltenen Färbungen auf. Die Behandlung des Materials erfolgt beispielsweise so, daß man das Wollgewebe oder die Wollflocke vor dem Färbeprozeß oder während desselben zusammen mit dem Farbstoff, beispielsweise einem Säurefarbstoff oder einem Reaktivfarbstoff, mit einer Verbindung der Formel (1), in einer Flotte gelöst bzw. in der erwähnten Färbeflotte selbst, behandelt. Die Einwirkung der Verbindungen der allgemeinen Formel (1) auf diese Fasermaterialien kann hierbei analog den Verfahrensbedingungen und Verfahrensweisen, wie sie allgemein üblich und in bekannter Weise zum Färben dieser Fasermaterialien mittels faserreaktiver Farbstoffe angewendet werden erfolgen (s. bspw. Seite 15, Zeilen 27 ff., bis Seite 16, Zeile 9, sowie Seite 16, Zeile 23 ff., bis Seite 17, Zeile 23, der deutschen Offenlegungsschrift Nr. 3 132 917).

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

**Beispiel 1**

a) 154 Teile Diphenyl werden innerhalb von etwa 90 Minuten in 760 Teile 100-%-iger Schwefelsäure bei einer Temperatur von etwa 30°C unter gutem Rühren eingetragen. Der Ansatz wird sodann etwa 1 Stunde zwischen 45 und 50°C weitergerührt und anschließend noch 2 Stunden bei 75°C. Sodann werden innerhalb von etwa 15 Stunden bei einer Temperatur von 65 bis 70°C langsam 1000 Teile Thionylchlorid hinzugegeben. Das Reaktionsgemisch wird nach Beendigung dieser Reaktion auf 40 bis 45°C abgekühlt und die Suspension auf 2400 Teile Eis eingerührt. 250 Teile Natriumchlorid werden zugegeben und die Temperatur bei 10°C gehalten. Es wird noch etwa 90 Minuten weitergerührt, das ausgefallene Produkt abgesaugt, mit insgesamt 1300 Teilen Wasser gewaschen und auf der Filternutsche (Filterpresse) trockengesaugt.
Ausbeute: 333 Teile entsprechend 95 % d.Th.;
Smp.: 203°C.

b) Das unter a) erhaltene Diphenyl-4,4'-disulfochlorid wird zur Sulfinsäure-Verbindung reduziert. Hierzu wird eine Lösung aus 325 Teilen einer 40-%-igen wäßrigen Natriumsulfit-Lösung und 280 Teilen einer wäßrigen 33-%-igen Natronlauge in 1660 Teilen Wasser hergestellt. In diese auf 80°C erwärmte und mit Natronlauge auf einen pH-Wert von 8 gestellte Lösung werden unter gutem Rühren das unter a) erhaltene Diphenyldisulfochlorid portionsweise innerhalb von etwa 90 Minuten eingetragen; hierbei wird der pH-Wert durch allmähliche Zugabe von etwa 430 Teilen einer 33-%-igen wäßrigen Natronlauge auf einen Wert von 8

gehalten. Man rührt noch etwa 1 Stunde bei dieser Temperatur nach, läßt sodann auf 20° C abkühlen, saugt das kristallin ausgefallene Diphenyl-natriumdisulfinat ab und trocknet es.
Ausbeute: etwa 318 Teile.

c) Das unter b) erhaltene Diphenyldisulfinat wird in 2500 Teilen Wasser bei einem pH-Wert zwischen 8,1 und 8,3 und bei einer Temperatur von etwa 60° C gelöst. Man leitet unter Konstanthaltung dieser Temperatur und dreses pH-Bereiches innerhalb von etwa 3 Stunden 132 Teilen Ethylenoxid in die Lösung ein; die Aufrechterhaltung dieses pH-Bereiches erfolgt mittels 20-%-iger wäßriger Phosphorsäure; der pH-Bereich wird auch noch beim anschließenden zweistündigen Nachrühren gehalten (insgesamt werden etwa 380 Teile 20-%-iger wäßriger Phosphorsäure verbraucht).

Das unter c) gebildete 4,4'-Bis-(β-hydroxyethylsulfonyl)-diphenyl fällt während der Oxethylierungsreaktion quantitativ aus. Nach Abkühlen des Reaktionsansatzes auf 20 bis 30° C wird es abgesaugt und mit Wasser nachgewaschen.
Ausbeute: 304 Teile entsprechend 82 % d.Th., bezogen auf das eingesetzte Diphenyl;
Smp.: 197° C; Reingehalt nach Analyse: größer als 98 %.
Analyse:

| | | | |
|---|---|---|---|
| ber.: | C 51,8 %, | H 4,8 %, | S 17,2 %; |
| gef.: | C 51,3 % | H 4,8 %, | S 17,3 %. |

**Beispiel 2**

In 1800 Teile 100-%-iger Schwefelsäure werden innerhalb von 2 Stunden bei anfänglich 0 bis 5° C, später bei maximal 30° C, 370 Teile 4,4'-Bis-(β-hydroxyethylsulfonyl)-diphenyl eingetragen. Der Ansatz wird noch 12 Stunden weitergerührt und sodann in 10 000 Teile Eiswasser eingerührt. Mittels etwa 1700 Teilen Calciumcarbonat wird ein pH-Wert von 5 eingestellt, das gebildete Calciumsulfat abgesaugt und mit 3000 Teilen Wasser nachgewaschen. Das Filtrat wird mit 600 Teilen Kaliumchlorid versetzt, und die so ausgesalzene erfindungsgemäße 4,4'-Bis-(β-sulfatoethylsulfonyl)-diphenyl-Verbindung abgesaugt und unter reduziertem Druck bei 30 bis 60° C getrocknet.

Man erhält ein farbloses, Kaliumchlorid-haltiges und wenig Kaliumsulfat-haltiges Produkt, das 450 Teile der erfindungsgemäßen Sulfatoverbindung in Form deren Kaliumsalz enthält.

Mit der erfindungsgemäßen Verbindung kann die Naßreißfestigkeit von Wolle und ebenso die Naßechtheiten von mit Farbstoffen erzielten Wollfärbungen verbessert werden, wenn man erfindungsgemäß dieses Material in einem die erfindungsgemäße Verbindung enthaltenden wäßrigen Bad vorbehandelt oder aber auch diese erfindungsgemäße Verbindung einem den Farbstoff enthaltenden Färbebad für Wolle zusetzt (vgl. auch das nachfolgende Beispiel 5).

**Beispiel 3**

Zur Herstellung der erfindungsgemäßen 4,4-Bis-(β-phosphatoethylsulfonyl)-diphenyl-Verbindung trägt man zu einer auf 100° C erwärmten Mischung aus 600 Teilen einer Polyphosphorsäure, die 84-%-ig an Phosphorpentoxid ist, und 108 Teilen einer 80-%-igen wäßrigen Phosphorsäure unter gutem Rühren innerhalb von 15 Minuten 370 Teile 4,4'-Bis-(β-hydroxyethylsulfonyl)-diphenyl ein. Man rührt die erhaltene Schmelze noch 2 Stunden bei 100° C nach und läßt sodann, ohne abzukühlen, 1400 Teile Wasser einlaufen, wobei die Temperatur 80° C nicht unterschreiten soll. Die so erhaltene Lösung wird noch etwa 30 Minuten unter Rückfluß gekocht, sodann unter Abkühlenlassen mehrere Stunden nachgerührt. Man versetzt die Lösung mit 100 Teilen Kaliumchlorid und rührt nochmals 2 Stunden nach.

Die ausgesalzene erfindungsgemäße 4,4'-Bis-(β-phosphatoethylsulfonyl)-diphenyl-Verbindung wird abgesaugt und unter reduziertem Druck bei 30 bis 60° C erhalten. Man erhält ein farbloses elektrolythaltiges (vorzugsweise Kaliumchlorid) Produkt, das 500 Teile der erfindungsgemäßen Phosphatoverbindung in Form ihres Kaliumsalzes enthält.

Mit der erfindungsgemäßen Verbindung kann die Naßreißfestigkeit von Wolle und ebenso die Naßechtheiten von mit Farbstoffen erzielten Wollfärbungen verbessert werden, wenn man erfindungsgemäß dieses Material in einem die erfindungsgemäße Verbindung enthaltenden wäßrigen Bad vorbehandelt oder aber auch diese erfindungsgemäße Verbindung einem den Farbstoff enthaltenden Färbebad für Wolle zusetzt; hierbei kann man analog dem nachfolgenden Beispiel 5 verfahren.

**Beispiel 4**

Zur Herstellung der erfindungsgemäßen 4,4'-Bis-(β-thiosulfatoethylsulfonyl)-diphenyl-Verbindung werden 545 Teile 4,4'-Bis-(β-sulfatoethylsulfonyl)-diphenyl in 4000 Teilen Wasser gelöst; die Lösung wird auf 40°C erwärmt und mit 800 Teilen einer 20-%-igen wäßrigen Natronlauge unter Rühren während 15 Minuten versetzt. Die Lösung wird noch 30 Minuten bei 40°C weitergerührt, und sodann werden 750 Teile kristallwasserhaltiges Natriumthiosulfat hinzugegeben, die Lösung auf 70 bis 75°C erwärmt und der pH mittels einer wäßrigen 50-%-igen Essigsäure auf einen Wert zwischen 5,7 und 6,2 gestellt. Man hält diesen pH-Bereich noch 3 Stunden mittels weiterer wäßriger Essigsäure.

Die erfindungsgemäße Thiosulfatoverbindung wird durch Zugabe von 600 Teilen Kaliumchlorid ausgesalzen und isoliert. Man erhält 480 Teile eines elektrolythaltigen Pulvers (vorwiegend Kaliumchlorid), das 300 Teile (entsprechend einer Ausbeute von 55 % d.Th.) des Kaliumsalzes von 4,4'-Bis-(β-thiosulfatoethylsulfonyl)-diphenyl enthält.

Die erfindungsgemäße Verbindung zersetzt sich langsam beim Erhitzen über 200°C.

Mit der erfindungsgemäßen Verbindung kann die Naßreißfestigkeit von Wolle und ebenso die Naßechtheiten von mit Farbstoffen erzielten Wollfärbungen verbessert werden, wenn man erfindungsgemäß dieses Material in einem die erfindungsgemäße Verbindung enthaltenden wäßrigen Bad vorbehandelt oder aber auch diese erfindungsgemäße Verbindung einem den Farbstoff enthaltenden Färbebad für Wolle zusetzt; hierbei kann man analog dem nachfolgenden Beispiel 5 verfahren.

**Beispiel 5** (Anwendung)

a) 100 g Wollgarn wurden in 2 l eines wäßrigen Bades, das 2 g des Säurefarbstoffes mit der Colour Index-Bezeichnung C.I. Acid Violet 9 (C.I.-Nr. 45 190) sowie 2 g Schwefelsäure und 10 g Natriumsulfat enthielt, zunächst 30 Minuten bei 100°C gefärbt. Sodann wurden 3 g der erfindungsgemäßen Verbindung 4,4'-Bis-(β-sulfatoethylsulfonyl)-diphenyl in Form des Natriumsalzes hinzugegeben, der pH-Wert des Färbebades auf 5 gestellt und der Färbeprozeß noch weitere 60 Minuten bei 100°C weitergeführt. Das gefärbte Gewebe wurde anschließend aus dem Bad herausgenommen, mit warmem und kaltem Wasser sowie intensiv gespült und anschließend getrocknet.

Das gefärbte Wollgarn wurde auf Farbechtheit in der Wäsche nach DIN 54 010 ausgeprüft. Der Farbton des Wollgarnes zeigte eine Farbtonänderung entsprechend der Bewertungsnote 4.

b) 100 g Wollgarn wurden in 2 l eines wäßrigen Bades, das 2 g des Säurefarbstoffes C.I. Acid Violet 9 (C.I.-Nr. 45 190) sowie 2 g Schwefelsäure und 10 g Natriumsulfat enthielt, 60 Minuten bei 100°C gefärbt. Das gefärbte Gewebe wurde anschließend aus dem Bad herausgenommen, mit warmem und kaltem Wasser intensiv gespült und anschließend getrocknet. Das gefärbte Wollgarn wurde auf Farbechtheit in der Wäsche nach DIN 54 010 ausgeprüft. Der Farbton des Wollgarnes zeigte eine Farbtonänderung entsprechend der Bewertungsnote 2.

**Patentansprüche**

1. Verbindung entsprechend der allgemeinen Formel (1)

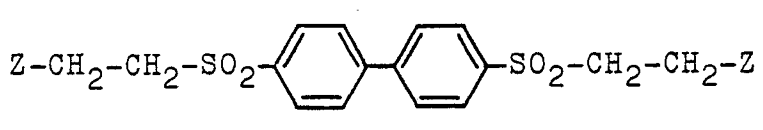

(1)

in welcher die Reste Z, zueinander gleich oder voneinander verschieden, jeder eine Hydroxygruppe oder eine Sulfatogruppe (entsprechend der allgemeinen Formel $-OSO_3M$, in welcher M für ein Wasserstoffatom oder ein Alkalimetall oder für das Äquivalent eines Erdalkalimetalls steht) oder eine Thiosulfatogruppe (entsprechend der allgemeinen Formel $-S-SO_3M$ mit M der obengenannten Bedeutung) oder eine Phosphatogruppe (entsprechend der allgemeinen Formel $-OPO_3M_2$ mit M der obengenannten Bedeutung) bedeuten.

2. Verbindung nach Anspruch 1, in welcher jedes Z dieselbe Bedeutung besitzt.

3. Verbindung nach Anspruch 1, in welcher jede Z eine Sulfato-, Thiosulfato- oder Phosphatogruppe bedeutet.

4. Verbindung nach Anspruch 1, in welcher jedes Z eine Sulfatogruppe ist.

5. Verfahren zur Herstellung einer Verbindung der in Anspruch 1 genannten allgemeinen Formel (1), in welcher jedes Z für eine Hydroxygruppe steht, dadurch gekennzeichnet, daß man Diphenyl zunächst durch Sulfochlorierung in Diphenyl-4,4'-disulfochlorid und dieses sodann mit einem Alkali- oder Erdalkalisulfit oder -hydrogensulfit in die Diphenyl-4,4'-disulfinsäure überführt und diese wiederum mittels Ethylenoxid zum 4,4'-

Bis-(β-hydroxyethylsulfonyl)-diphenyl umsetzt.

6. Verfahren zur Herstellung einer Verbindung der in Anspruch 1 genannten allgemeinen Formel (1), in welcher eines oder beide Z, vorzugsweise beide Z, jedes eine Sulfato- oder eine Phosphatogruppe bedeuten, dadurch gekennzeichnet, daß man 4,4'-Bis-(β-hydroxyethylsulfonyl)-diphenyl mit einem Sulfatierungsmittel bzw. Phosphatierungsmittel umsetzt.

7. Verfahren zur Herstellung von 4,4'-Bis-(β-thiosulfatoethylsulfonyl)-diphenyl, dadurch gekennzeichnet, daß man 4,4'-Bis-(β-sulfatoethylsulfonyl)-diphenyl in wäßriger Lösung zunächst bei einem pH-Wert zwischen 10 und 13 und bei einer Temperatur zwischen 20 und 80°C in die Vinylsulfonylverbindung überführt und diese sodann ohne Zwischenisolierung bei einem pH-Wert von 4,5 bis 7,5 und einer Temperatur zwischen 40 und 90°C mit einem Alkalithiosulfat umsetzt.

## Claims

1. A compound of the general formula (1)

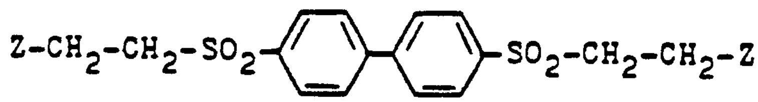 (1)

in which the radicals Z, which are identical to or different from one another, each denote a hydroxyl group or a sulfato group (conforming to the general formula $-OSO_3M$, in which M stands for a hydrogen atom or an alkali metal or for one equivalent of an alkaline earth metal) or a thiosulfato group (conforming to the general formula $-S-SO_3M$, where M has the abovementioned denotation) or a phosphato group (conforming to the general formula $-OPO_3M_2$, where M has the abovementioned denotation).

2. A compound as claimed in claim 1, in which each Z has the same denotation.

3. A compound as claimed in claim 1, in which each Z denotes a sulfato, thiosulfato or phosphato group.

4. A compound as claimed in claim 1, in which each Z is a sulfato group.

5. A process for preparing a compound of the formula (1), mentioned in claim 1, in which each Z stands for a hydroxyl group, which comprises first converting biphenyl by chlorosulfonation into biphenyl-4,4'-disulfonyl dichloride and then converting the latter with an alkali metal sulfite or alkaline earth metal sulfite or hydrogensulfite into biphenyl-4,4'-disulfinic acid and reacting the latter in turn with ethylene oxide to form 4,4'-bis-(β-hydroxyethylsulfonyl)-biphenyl.

6. A process for preparing a compound of the formula (1), mentioned in claim 1, in which one or both Z, preferably both Z, each denote a sulfato or phosphato group, which comprises reacting 4,4'-bis-(β-hydroxyethylsulfonyl)-biphenyl with a sulfating agent or with a phosphating agent.

7. A process for preparing 4,4'-bis-(β-thiosulfatoethylsulfonyl)-biphenyl, which comprises first converting 4,4'-bis-(β-sulfato-ethylsulfonyl)-biphenyl in aqueous solution at a pH between 10 and 13 and at a temperature between 20 and 80°C into the vinylsulfonyl compound and then reacting the latter without intermediate isolation at a pH of 4.5 to 7.5 and at a temperature between 40 and 90°C with an alkali metal thiosulfate.

## Revendications

1. Composé répondant à la formule générale

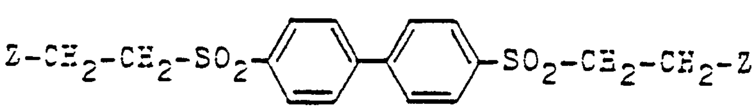 (1)

dans laquelle les symboles Z, qui peuvent avoir la même signification ou des significations différentes, représentent chacun un radical hydroxy, un radical sulfato (répondant à le formule générale $-OSO_3M$ dans laquelle M représente un atome d'hydrogène ou un métal alcalin ou l'équivalent d'un métal alcalino-terreux), un radical thiosulfato (répondant à la formule générale $-S-SO_3M$ dans laquelle M a la signification indiquée ci-dessus) ou un radical phosphato (répondant à la formule générale $-OPO_3M_2$ dans laquelle M a la signification indiquée ci-dessus).

2. Composé selon la revendication 1 dans lequel les Z ont la même signification.

3. Composé selon la revendication 1 dans lequel les Z représentent chacun un radical sulfato, thiosulfato- ou phosphato.

4. Composé selon la revendication 1 dans lequel les Z représentent chacun un radical sulfato.

5. Procédé pour préparer un composé qui répond à la formule générale (1) selon la revendication 1 et dans

lequel les Z représentent chacun un radical hydroxy, procédé caractérisé en ce qu'on transforme d'abord le biphényle, par chlorosulfonylation, en bis-(chlorosulfonyl)-4,4' biphényle, puis celui-ci, au moyen d'un sulfite ou d'un hydrogénosulfite de métal alcalin ou de métal alcalino-terreux, en l'acide biphényle-disulfinique-4,4', et on fait réagir ce dernier avec l'oxyde d'éthylène pour le transformer en le bis-(hydroxy-2 éthylsulfonyl)-4,4' biphényle.

6. Procédé pour préparer un composé qui répond à la formule générale (1) selon la revendication 1 et dans lequel l'un des symboles Z ou, mieux, chacun d'eux représente un radical sulfato ou phosphato, procédé caractérisé en ce qu'on fait réagir le bis-(hydroxy-2 éthylsulfonyl)-4,4' biphényle avec un agent de sulfatation ou un agent de phosphatation.

7. Procédé pour préparer le bis-(thiosulfato-2 éthylsulfonyl)-4,4' biphényle, procédé caractérisé en ce qu'on transforme d'abord le bis-(sulfato-2 éthylsulfonyl)-4,4' biphényle, en solution aqueuse, à un pH compris entre 10 et 13 et à une température comprise entre 20 et 80° C, en le composé vinylsulfonylique, puis on fait réagir celui-ci, sans l'isoler intermédiairement, à un pH compris entre 4,5 et 7,5 et à une température comprise entre 40 et 90° C, avec un thiosulfate de métal alcalin.